# EUROPÄISCHE PATENTSCHRIFT

(11) **EP 1 576 147 B1**
(45) Veröffentlichungstag und Bekanntmachung des Hinweises auf die Patenterteilung: **24.05.2006**
(21) Anmeldenummer: 03780150.3
(22) Anmeldetag: 12.12.2003
(51) Int. Cl.: C12N 1/20, C12N 15/01

(54) **BAKTERIENSTÄMME DELFTIA ACIDOVORANS MC1-S, IHRE HERSTELLUNG SOWIE IHRE VERWENDUNG ZUR PRODUKTION DER S-ENANTIOMEREN VON 2-PHENOXYPROPIONSÄURE-DERIVATEN**
BACTERIAL STRAINS DELFTIA ACIDOVORANS MC1-S, PRODUCTION AND USE THEREOF FOR THE PRODUCTION OF THE S ENANTIOMERS OF 2-PHENOXYPROPIONIC ACID DERIVATIVES
SOUCHES BACTERIENNES DELFTIA ACIDOVORANS MC1-S, LEUR PRODUCTION ET LEUR UTILISATION POUR PRODUIRE LES ENANTIOMERES S DE DERIVES D'ACIDE 2-PHENOXYPROPIONIQUE

(30) Priorität: 18.12.2002 DE 10260457
(43) Veröffentlichungstag der Anmeldung: 21.09.2005
(73) Patentinhaber: UFZ-UMWELTFORSCHUNGSZENTRUM Leipzig-Halle GmbH, 04318 Leipzig (DE)
(72) Erfinder: MÜLLER, Roland, 04425 Taucha (DE); NEYTSCHEV, Monika, 04328 Leipzig (DE); SCHUMANN, Cornelia, 04315 Leipzig (DE)
(74) Vertreter: Rasch, Dorit
(86) Internationale Anmeldenummer: PCT/EP2003/014152
(87) Internationale Veröffentlichungsnummer: WO 2004/055172

(56) Entgegenhaltungen:
- MUELLER R H ET AL: "Comamonas acidovorans strain MC1: A new isolate capable of degrading the chiral herbicides dichlorprop and mecoprop and the herbicides 2,4-D and MCPA" MICROBIOLOGICAL RESEARCH, FISCHER, JENA,, DE, Bd. 154, Nr. 3, Dezember 1999 (1999-12), Seiten 241-246, XP009026769 ISSN: 0944-5013
- WESTENDORF A ET AL: "THE TWO ENANTIOSPECIFIC DICHLORPROP/ALPHA-KETOFLURARATE-DIOXYGENAS ES FROM DELFTIA ACIDOVORANS MC1: PROTEIN AND SEQUENCE DATA OF RDPA AND SDPA" MICROBIOLOGICAL RESEARCH, FISCHER, JENA,, DE, Bd. 157, Nr. 4, 2002, Seiten 317-322, XP009026602 ISSN: 0944-5013
- MUELLER R H ET AL: "SEPARATION OF TWO DICHLORPROP/ALPHA-KETOGLUTARATE DIOXYGENASES WITH ENANTIOSPECIFIC PROPERTIES FROM COMAMONAS ACIDOVORANS MC1" ACTA BIOTECHNOLOGICA, AKADEMIE VERLAG, BERLIN, DE, Bd. 19, Nr. 4, 1999, Seiten 349-355, XP009026661 ISSN: 0138-4988
- WESTENDORF A ET AL: "PURIFICATION AND CHARACTERIZATION OF THE ENANTIOSPECIFIC DIOXYGENASES FROM DELFTIA ACIDOVORANS MC1 INITIATING THE DEGRADATION OF PHENOXYPROPIONATE AND PHENOXYACETATE HERBICIDES" ACTA BIOTECHNOLOGICA, AKADEMIE VERLAG, BERLIN, DE, Bd. 23, Nr. 1, 2003, Seiten 3-17, XP009026596 ISSN: 0138-4988
- HOFFMANN DOREEN ET AL: "A transposon encoding the complete 2,4-dichlorophenoxyacetic acid degradation pathway in the alkalitolerant strain Delftia acidovorans P4a." MICROBIOLOGY (READING), Bd. 149, Nr. 9, September 2003 (2003-09), Seiten 2545-2556, XP002274427 ISSN: 1350-0872 (ISSN print)

## Beschreibung

Die Erfindung betrifft neue Bakterienstämme *Delftia acidovorans* MC1-S, Verfahren zu ihrer Herstellung sowie ihre Verwendung zur Produktion von hochreinen S-Enantiomeren von 2-Phenoxypropionsäure-Derivaten. Insbesondere betrifft sie den Bakterienstamm *Delftia acidovorans* MC1-S DSM 15377 sowie seine Verwendung.

Asymmetrische Synthesen sind von großer Bedeutung für die künftige Forschung und Anwendung, insbesondere im Bereich der Fein- bzw. Biochemikalien, der Arznei- und Pflanzenschutzmittel, der Geschmacks- und Riechstoffe etc. und damit für die Volkswirtschaft überhaupt.

Für die Gewinnung optischer Antipoden aus racemischen Ausgangsverbindungen wurde in der Zeit, als α-Aryloxyalkylcarboxylate als Herbizide entdeckt und hinsichtlich ihrer enantiospezifischen Wirkung näher untersucht wurden, die selektive Kristallisation über optisch aktive Lösungsmittel als eine praktikable Methode zur Anreicherung entsprechender Enantiomere genutzt (Matell 1953, Arkiv Kemi 6, 355). Für die asymetrische Synthese von Enantiomeren hat sich der Austausch von primären bzw. sekundären Hydroxygruppen in Alkoholen R-OH durch H-X in Gegenwart von trivalenten Phosphorverbindungen und Azodicarboxylat-Derivaten als geeignet erwiesen (Mitsunobu 1967, Bull. Chem. Soc. Japan 40, 2380). Die Reaktion erfolgt unter milden Bedingungen und die Ausbeuten sind im wesentlichen gut. Bei dieser Reaktion erfolgt eine komplette Inversion der Konfiguration der enantiomeren alkoholischen Ausgangsverbindungen. Dieser Reaktionstyp hat sich durch Variation von R-OH (Mitsunobu et al. 1973, Bull. Chem. Soc. Japan 46, 2833) und H-X (Loibner und Zhibal 1976, Helv. Chim. Acta 59, 2100) als sehr variabel einsetzbar gezeigt. Auf diese Weise konnten unter Nutzung von 2,4-Dichlorphenol bzw. 4-Chlor-2-methylphenol und (R)- bzw. (S)-Allyllacetat in Gegenwart von Triphenylphosphin und Diethylazodicarboxylat enantiomere Phenoxypropionat-Verbindungen ((S)-2,4-DP und (R) -2,4-DP bzw. (S) -MCPP und (R) - MCPP) synthetisiert werden. Die enantiomere Selektivität der Reaktion (*enantiomeric excess, ee*) ist entsprechend hoch und bewegt sich bei Werten >90% (Zipper 1998; Diss. ETH Zürich No. 12543). Für viele Anwendungen genügt dieser ee. Für die Nutzung als Fein- bzw. Biochemikalien dagegen ist diese enantiomere Reinheit nicht ausreichend.

Der Wildtyp *Delftia acidovorans* MC1 (Müller et al. 1999, Microbiol. Res. 154, 241) ist bekanntermaßen zum kompletten Abbau von racemischen 2-Phenoxypropionaten befähigt, z.B. von (RS)-2,4-DP, (RS)-MCPP, (RS)-2-(m-Chlorphenoxy)propionat oder (RS)-2-(4-Chlorphenoxy)propionat) (Müller and Babel 1999, Acta Biotechnol. 19, 349; Müller et al. 2001, Microbiol. Res. 156, 121). Der Wildtyp-Stamm MC1 verfügt über zwei spezifische Enzyme, welche wesentlicher Bestandteil sind, nämlich das Enzym (R)-2,4-DP/α-Ketoglutarat-Dioxygenase (RdpA) und das Enzym (S)-2,4-DP/α-Ketoglutarat-Dioxygenase (SdpA). Über die Organisation der betreffenden Gene weiß man bislang nur, dass sie offensichtlich nicht auf einem Plasmid lokalisiert sind.

Der Stamm MC1 gilt als stabil und leistungsaktiv gegenüber hohen Herbizidkonzentrationen, die bis zur lösungsmittelbedingten Grenze reichen und in Abhängigkeit vom pH-Wert Werte von 5 bis 10 mM betragen können. Andere Stämme wie *Alkaligenes eutrophus (Ralstonia eutropha)* JMP134, die ebenfalls zum Herbizidabbau in der Lage sind, sind wesentlich instabiler und schon bei Konzentrationen von 0,5 mM Herbizid in ihrer Aktivität stark eingeschränkt (Müller et al. 1997, Appl. Microbiol. Biotechnol. 48, 648).

Wenn der Stamm *Delftia acidovorans* MC1 auch leistungsaktiv gegenüber hohen Herbizidkonzentrationen ist, zeichnet er sich andererseits als Wildtyp durch eine extreme genetische Instabilität für die Verwertung von Phenoxyalkansäuren aus. Unter nicht selektiven Bedingungen führt die Kultivierung des Wildtyps *Delftia acidovorans* MC1 innerhalb von 5-10 Generationen zu einem Totalverlust der Fähigkeit zur Verwertung von (2,4-Dichlorphenoxy)essigsäure (2,4-D) und 2-(2,4-Dichlorphenoxy)propionsäure [(RS)-2,4-DP]. Als Ursache wird der Verlust der Aktivität zur Spaltung der Etherbindung, katalysiert durch die in Rede stehenden substrat- und stereospezifisch wirkenden α-Ketoglutarat-abhängigen Dioxygenasen, als RdpA (mit Spezifität für R-Enantiomere) und SdpA (mit Spezifität für S-Enantiomere) bezeichnet, angesehen. Der Verlust der Verwertungsaktivität betrifft die beiden dafür zuständigen Gene gleichermaßen, d.h. man erhält in der Regel doppelt negative Mutanten.

Bislang konnten keine Organismen nachgewiesen werden, die sich zur Racemat-Spaltung und Herstellung von reinen Enantiomeren von 2-Phenoxypropionsäure-Derivaten eignen.

Der Erfindung lag deshalb die Aufgabe zugrunde, Organismen zu suchen und bereitzustellen, die zur Gewinnung von hochreinen S-Enantiomeren von 2-Phenoxypropionsäure-Derivaten geeignet sind.

Die Aufgabe der Erfindung konnte durch Mutanten des Stammes *Delftia acidovorans* MC1 gelöst werden. Diese neuen Bakterienstämme, genannt MC1-S, sind in der Lage S-Enantiomere hochrein zu produzieren und exprimieren die Aktivitäten zur Spaltung und Verwertung der R-Enantiomeren konstitutiv. Nach Induktion der Chlorphenol-Hydroxylase und Bildung der entsprechenden Catechole und nach Induktion der Enzyme des modifizierten ortho-Wegs (Abbau über den an sich bekannten Chlorcatechol-Weg der Bakterien mittels ringspaltender Dioxygenasen) sind die erfindungsgemäßen Bakterienstämme MC1-S zum vollständigen Abbau der R-Enantiomeren unter Bildung von Wasser, CO₂ und Salzsäure befähigt und damit zur hochreinen Produktion des S-Enantiomeren.

Die Bakterienstämme *D. acidovorans* MC1-S sind stabil gegenüber 2-Phenoxypropionsäure-Derivaten und stoffwechselaktiv bis zur Löslichkeitskonzentration der entsprechenden Racemate bei pH-Werten bis 9,0.

Als besonders bevorzugt zur Produktion von S-Enantiomeren von 2-Phenoxypropionsäure-Derivaten hat sich der Stamm MC1-S erwiesen, der am 17.12.2002 bei der Deutschen Sammlung von Mikroorganismen und Zellkulturen GmbH unter der Nummer DSM 15377 hinterlegt wurde.

Die Erzeugung der neuen Stämme MC1-S erfolgte durch Kultivierung des Wildtyp-Stammes *Delftia acidovorans* MC1 mit (2,4-Dichlorphenoxy)essigsäure (2,4-D) als selektivem Substrat und als alleiniger Kohlenstoff- und Energiequelle. Es wurden überraschend Klone mit leistungsstabiler Ausprägung der Herbizidverwertungsaktivität mit ausschließlicher Aktivität für R-Enantiomere von (RS)-2-Phenoxypropionsäure-Derivaten erhalten, die in der Lage sind, die S-Enantiomeren der 2-Phenoxypropionsäure-Derivate hochrein zu produzieren. Das ist insofern überraschend, da 2,4-D vom Wildstamm MC1 eher schlecht verwertet wird und das Substrat maximale Wachstumsraten von 0,05 h⁻¹ zulässt.

In einer bevorzugten Ausführungsvariante erfolgt die Kultivierung bei Durchflussraten im Bereich von größer Null bis 0,3 h⁻¹, vorzugsweise in einem an sich üblichen Nährmedium. Neben 2,4-D dienen gegebenfalls weitere Substrate als Kohlenstoff- und Energiequelle, vorzugsweise Succinat oder Pyruvat.

In definierten zeitlichen Abständen wird die Durchflussrate von 0,05 h⁻¹ auf 0,3 h⁻¹ gesteigert und über einen längeren Zeitraum auf dem höchsten Wert konstant gehalten. Dieser Vorgang wird mehrmals wiederholt. Die stationäre 2,4-D-Konzentration stieg während des Geschwindigkeitsgradienten an. Die Wachstumseigenschaften von Klonen aus dieser Kultur werden mit (RS)-2,4-Dichlorphenoxypropionsäure (2,4-DP) als selektivem Substrat verfolgt. Stark wachsende Kolonien werden z.B. über Flüssigkultur in Komplexmedium weiter vermehrt und anschließend erneut selektiert.

Die Kultivierung erfolgt bevorzugt bei pH-Werten von 8 bis 9, vorzugsweise 8,5 und Temperaturen von 20 bis 35°C, vorzugsweise 30°C, über einen Zeitraum von ca. 2 bis 8 Wochen.

Besonders bevorzugt wird *Delftia acidovorans* MC1 bei einer Durchflussrate (D) von 0,1 h⁻¹ in Gegenwart von 2,4-D und Succinat über einen Zeitraum von 10 Tagen kontinuierlich kultiviert. Anschließend wird die Durchflussrate mit einem ΔD von 0,06 h⁻¹ und einem Zeitintervall Δt von 1,5 h schrittweise auf eine Rate von 0,24 h⁻¹ gesteigert und weitere drei Tage bei dieser Wachstumsgeschwindigkeit kultiviert. Stark wachsende Kolonien werden selektiert, über Flüssigkultur in Komplexmedium vermehrt und anschließend erneut kultiviert. Diese Prozedur wird bis zu dreimal wiederholt. Klone von stark gewachsenen Kolonien werden auf die selektiven Substrate (R)-2,4-DP, (S)-2,4-DP und 2,4-D aufgebracht. Über mehrere Passagen stark wachsende Kolonien werden für Enzymtests auf RdpA und SdpA verwendet.

Neben Klonen, die nach wie vor Aktivität für (RS)-Derivate zeigen, werden überraschend Bakterienstämme MC1-S erhalten und selektiert, die ausschließliche Aktivität zur Verwertung der R-Enantiomeren zeigen. Offensichtlich besitzen sie demzufolge ausschließliche Aktivität für das Enzym RdpA und sind SdpA negativ. Klone, die ausschließlich Aktivität für SdpA besitzen, wurden nicht gefunden.

Zum Nachweis der Stabilität der Leistung wurden die erfindungsgemäßen Bakterienstämme unter nicht selektiven Bedingungen vermehrt. Im Unterschied zum Wildtyp-Stamm MC1 sind sie genetisch stabil. Über eine beobachtete Spanne von 80 Generationen konnte Leistungsstabilität beobachtet werden. Die erfindungsgemäßen Stämme MC1-S sind zur 100%igen Spaltung von R-Enantiomeren geeignet.

Die Klone mit Aktivität für (RS)-Derivate erwiesen sich als genetisch leistungsstabile Mutanten. Erfingungsgemäße Stämme *D. acidovorans* MC1-S können auch durch genetische Veränderung des Wildtyps *D. acidovorans* MC1, insbesondere unter Nutzung der genetisch leistungsstabilen Mutanten erhalten werden, indem das Gen *sdpA* für das Enzym (S)-2,4-DP/ α-Ketoglutarat-Dioxygenase (SdpA), das spezifisch für die Spaltung von S-Enantiomeren der Phenoxypropionate ist, abgeschaltet bzw. eliminiert wird. Durch eine eventuelle zusätzliche Vervielfältigung (Gendosis-Effekt) von *rdpA,* das die (R)-2,4-DP/α-Ketoglutarat-Dioxygenase codiert, kann eine Steigerung der Enzymmenge RdpA erreicht werden.

Racemische 2-Phenoxypropionsäure-Derivate im Sinne der Erfindung sind bevorzugt
2-(2,4-Dichlorphenoxy)propionsäure [(RS)-2,4-DP],
2-(4-Chlor-2-methylphenoxy)propionsäure [(RS)-MCPP] und
2-(m-Chlorphenoxy)propionsäure bzw.
2-(4-Chlorphenoxy)propionsäure.

Zur erfindungsgemäßen Gewinnung hochreiner S-Enantiomere von (RS)-2-Phenoxypropionsäure-Derivaten unter Verwendung der Stämme *D. acidovorans* MC1-S werden racemische Gemische von entsprechenden 2-Phenoxypropionaten als Ausgangssubstrate und als alleinige Kohlenstoff- und Energiequelle eingesetzt. Ggf. erfolgt die Nutzung an sich üblicher Nährmedien mit einer weiteren Kohlenstoff- und Energiequelle. Die Kultivierung erfolgt kontinuierlich oder diskontinuierlich bei pH-Werten von 6,5 bis 10,0, vorzugsweise zwischen 8,0 und 9,5 und bei Temperaturen zwischen 15°C und 37°C, vorzugsweise bei 20°C bis ca. 30°C. Die Biomasse wird abgetrennt und das jeweils nicht metabolisierte Enantiomer durch Säurefällung und ggf. nach zusätzlichen Reinigungs- und Waschschritten, gewonnen. Die Fällung erfolgt bevorzugt mit HCl bei einem pH Wert von 1,5; die Abtrennung des Fällungsproduktes erfolgt vorzugsweise durch Zentrifugation.

In besonders bevorzugten Varianten der Erfindung werden zur Herstellung hochreiner S-Enantiomerer als Ausgangssubstrate (RS)-2,4-DP, (RS)-MCPP und (RS)-2-(m-Chlorphenoxy) propionsäure, (RS)-2-(4-Chlorphenoxy)propionsäure eingesetzt, wodurch reines (S)-2,4-DP, reines (S)-MCPP und reine (S)-2-(m-Chlorphenoxy)propionsäure bzw. (S)-2-(4-Chlorphenoxy) propionsäure gewonnen werden.

Die Fermentation erfolgt nach an sich bekannten Techniken, wobei die Kultivierung des jeweiligen Stammes kontinuierlich oder diskontinuierlich durchgeführt werden kann.

Das kontinuierliche Fermentationsverfahren ist dadurch gekennzeichnet, dass man den jeweiligen erfindungsgemäßen Bakterienstamm bei bevorzugten Durchflussraten von D = 0,05 h⁻¹ bis 0,20 h⁻¹, besonders bevorzugt bei einer Durchflussrate von 0,06 h⁻¹ bis 0,15 h⁻¹, und einem bevorzugten pH-Wert zwischen 8,0 und 9,5 und einer bevorzugten Temperatur von Raumtemperatur bis ca. 30°C in Gegenwart des jeweiligen (RS)-2-Phenoxypropionsäure-Derivates als alleiniger Kohlenstoff- und Energiequelle und bevorzugt in Gegenwart eines mineralischen Mediums kultiviert. Vorzugsweise wird zur Gewährleistung einer vollständigen Umsetzung der R-Enantiomeren im Fermenterablauf erst nach einer gewählten Inkubationszeit unter aeroben Bedingungen (bevorzugt mindestens 1 Stunde) das Verfahren abgeschlossen.

Das erfindungsgemäße Fermentationsverfahren zur Gewinnung von reinen S-Enantiomeren kann auch diskontinuierlich erfolgen, wobei in die Erzeugung der Biomasse ein Wachstumsschritt unter Nutzung eines an sich üblichen Nährmediums mit einer Kohlenstoff- und Energiequelle mit einer anschließenden Konditionierung des Stammes eingeschlossen sein kann. Dazu wird der Stamm zuerst in einem Nährmedium, vorzugsweise Pyruvat, in einer bevorzugten Menge von 2 bis 5 g/l, kultiviert. Als Inokulum werden Kolonien nach Wachstum auf einem der (RS)-2-Phenoxypropionsäure-Derivate eingesetzt. Das heißt, nach Erreichen der stationären Phase wird die Biomasse durch Zugabe eines (RS)-2-Phenoxypropionsäure-Derivates (vorzugsweise 0,5 mM (RS)-2-Phenoxypropionsäure-Derivat) induziert und in ein Medium, das die Biomasse in einer bevorzugten Konzentration von 0,1 bis 0,5 g/l mit bevorzugten pH-Werten von 8 bis 9,5 und das (RS)-2-Phenoxypropionsäure-Derivat in Abhängigkeit von der Temperatur in einer Konzentration von vorzugsweise 5 mM (bei Raumtemperatur, 18-25°C) bis 10 mM (bei 30°C) enthält, überführt (mineralisches Medium wie oben). Dabei kann die Biomasse als vorliegende Suspension oder nach vorheriger Antrennung eingesetzt werden.

In einer alternativen bevorzugten Ausführungsvariante der Kultivierung kann der Bakterienstamm kontinuierlich oder diskontinuierlich auf einem (RS)-2-Phenoxypropionsäure-Derivat als alleiniger Kohlenstoff- und Energiequelle angezogen werden. Die so gewonnene Biomasse wird in ein Medium überführt, wobei die Biomasse in einer bevorzugten Konzentration von 0,1 bis 0,5 g/l bei einem bevorzugten pH-Wert von 8 bis 9,5 vorliegt und das Medium das (RS)-2-Phenoxypropionsäure-Derivat in Abhängigkeit von der Temperatur in einer Konzentration von vorzugsweise 5 mM (bei Raumtemperatur) bis 10 mM (bei 30°C) enthält.

Die Aufbereitung des Fällungsproduktes (des S-Enantiomeren) erfolgt vorzugsweise durch ggf. mehrfaches Waschen mit HCl (bevorzugt 0,5 n HCl), Trocknung, ggf. mehrfaches Extrahieren und Umkristallisieren in einem Ether, bevorzugt in Diethylether.

Unter diesen Bedingungen erfolgt eine quantitative Degradation des R-Enantiomeren unter Wandlung in bakterielle Biomasse, Kohlendioxid, HCl und Wärme und das Zurückbleiben des nicht verstoffwechselten S-Enantiomeren.

Die Konzentration des abgereicherten R-Enantiomeren war in Abhängigkeit von der Biomassekonzentration in einem Zeitraum von 6 bis 24 Stunden auf einen über HPLC nicht mehr nachweisbaren Wert abgesunken. Die Ausbeute des R-Enantiomeren kann prinzipiell mit 100% angenommen werden und reduziert sich nur um die im Zuge der Aufarbeitung anfallenden Verluste.

Neben der Gewinnung des reinen S-Enantiomeren kann der jeweilige Stamm auch angewandt werden, um kommerzielle S-Enantiomere Produkte aufzubessern und hochzureinigen, d.h. störende Verunreinigungen durch das entsprechende R-Enantiomer zu eliminieren. S-Enantiomere der betreffenden Phenoxyalkanoate sind derzeit nicht auf dem Markt.

Die erfindungsgemäßen Stämme MC1-S, insbesondere der Stamm DSM 15377, sind zur Produktion von Feinchemikalien einsetzbar und erschließen neue Produkte. Die Stämme sind darüber hinaus prinzipiell geeignet, das nicht gewünschte Enantiomer aus racemischen Gemischen durch produktiven Abbau auch im *bulk production* Maßstab zu eliminieren bzw. abzureichern und somit z.B. das als Herbizid nicht wirksame R-Enantiomer zu entfernen.

Die Verwendung der Stämme, insbesondere des Stammes MC1-S DSM 15377, vermeidet die Notwendigkeit, enantiomere Vorstufen/Verbindungen bei der Synthese des reinen S-Enantiomeren einsetzen zu müssen, die ihrerseits wieder aus speziellen Synthesen bzw. Reinigungsverfahren hervorgehen und damit entsprechend kostenintensiv sind. Statt dessen kann auf eine robuste und kostengünstige Synthese gesetzt werden.

Anschließend wird die Erfindung an Ausführungsbeispielen näher erläutert.

### Beispiel 1

### a) Kultivierung des Wildtyp-Stammes MC1

Der Stamm *Delftia acidovorans* MC1 wird auf einem mineralischen Medium folgender Zusammensetzung (in mg/l) NH₄Cl, 760; KH₂PO₄, 340; K₂HPO₄, 485; CaCl₂ * 6 H₂O, 27; MgSO₄ * 7 H₂O, 71.2; FeSO₄ * 7 H₂O, 4.98; CuSO₄ * 5 H₂O, 0.785; MnSO₄ * 4 H₂O , 0.81; ZnSO₄ * 7 H₂O, 0.44; Na₂MoO₄ * 2 H₂O , 0.25) und 10 mM (RS)-2-(2,4-Dichlorphenoxy)propionate [(RS)-2,4-DP] als alleiniger Kohlenstoff- und Energiequelle kultiviert. Als Inokulation dienten auf (RS)-2,4-DP-Agarplatten gewachsene Kolonien. Die kontinuierliche Kultivierung erfolgte nach einer batch-Phase auf 2 mM (RS)-2,4-DP mit Durchflußraten im Bereich von D = 0.05 h⁻¹ bis D = 0.14 h⁻¹. Bevorzugt werden pH-Werte von 8,5 und Temperaturen von 30°C. Unter diesen Bedingungen ist die stationäre Konzentration von (R)-2,4-DP bzw. (S)-2,4-DP entsprechend der kinetischen Parameter für das Wachstum gering. Der pH-Wert kann im Bereich von 7,0 bis 9,0 variiert werden, ohne das Wachstum deutlich zu beeinflussen.

### b) Gewinnung von MC1-S-Stämmen

*D. acidovorans* MC1 wurde bei einer Durchflussrate (D) von 0,1 h⁻¹ über einen Zeitraum von 10 Tagen kontinuierlich auf 18 mM Succinat und 6 mM 2,4-D kultiviert. Anschließend wurde die Durchflussrate mit einem ΔD von 0,06 h⁻¹ und einem Zeitintervall Δt von 1,5 h schrittweise auf eine Rate von 0,24 h⁻¹ gesteigert und weitere drei Tage bei dieser Wachstumsgeschwindigkeit kultiviert. Die stationäre 2,4-D Konzentration stieg während des Geschwindigkeitsgradienten von 0,35 mM auf 2,7 mM an. Die Wachstumseigenschaften von Klonen aus dieser Kultur wurden über Agarplatten mit (RS)-2,4-DP als selektivem Substrat verfolgt. Stark wachsende Kolonien wurden selektiert, über Flüssigkultur in Komplexmedium vermehrt und anschließend auf Selektivplatten erneut kultiviert. Diese Prozedur wurde bis zu dreimal wiederholt. Klone von stark gewachsenen Kolonien wurden auf Agarplatten mit den selektiven Substrate (R)-2,4-DP, (S)-2,4-DP und 2,4-D aufgebracht. Davon wurden jeweils einzelne Klone von stark gewachsenen Kolonien auf Agarplatten mit den betreffenden Substraten über weitere Passagen auf Agarplatten mit (R)-2,4-DP, (S)-2,4-DP und 2,4-D als selektivem Substrat vermehrt. Am Ende der Prozedur wurden auf den einzelnen Substraten mit als stark wachsend ausgewählte Kolonien, die sich über mehrere Passagen als solche erwiesen hatten, Enzymtests auf RdpA und SdpA durchgeführt; Klone mit ausschließlicher Aktivität für RdpA (SdpA negativ) wurden selektiert. Als bevorzugter Stamm hat sich der unter DSM 15377 hinterlegte Stamm MC1-S erwiesen.

### Beispiel 2

### Produktion von S-2,4-DP

Der Stamm *Delftia acidovorans* MC1-S DSM 15377 wurde bei einer Durchflußrate von D=0.075 h⁻¹ auf einem mineralischen Medium folgender Zusammensetzung (in mg/l) NH₄Cl, 760; KH₂PO₄, 340; K₂HPO₄, 485; CaCl₂ * 6 H₂O 27; MgSO₄ * 7 H₂O, 71.2; FeSO₄ * 7 H₂O 4.98; CuSO₄ * 5 H₂O 0.785; MnSO₄ * 4 H₂O 0.81; ZnSO₄ * 7 H₂O 0.44; Na₂MoO₄ * 2 H₂O , 0.25) und 7,5 mM (RS)-2-(2,4-Dichlorphenoxy)propionate [(RS)-2,4-DP] als alleiniger Kohlenstoff- und Energiequelle bei pH 8,5 kontinuierlich kultiviert. Die stationären Substratkonzentrationen im Fermentationsablauf betrugen rund 3,65 mM (S)-2,4-DP und 0,05 mM (R)-2,4-DP. Der Fermenterablauf wurde unter aseptischen Bedingungen bzw. unter ungeschützten Bedingungen in einer Stickstoffatmosphäre in Portionen gesammelt und eine weitere Behandlung für mindestens 1 h bei Raumtemperatur zum Abbau des restlichen (R)-2,4-DP weiter belüftet. Die Biomasse wurde mittels Zentrifugation abgetrennt und der Überstand mit 1n HCl auf pH 1,5 gebracht. Das gefällte (S)-2,4-DP wurde ebenfalls mittels Zentrifugation abgetrennt, das Pellet 2 mal mit 0,5n HCl gewaschen und luft- bzw. vakuumgetrocknet. Die Gesamtausbeute an (S)-2,4-DP nach der Fermentation betrug ca. 100%, nach der Säure-Fällung reduziert sich die Ausbeute um die entsprechend der Löslichkeit der freien Säure in der wäßrigen Phase verbleibende Menge. Alternativ erfolgte die Aufarbeitung der gesamten Säure-Fällung oder des in Lösung verbleibenden Restes durch Lyophilisierung. Für eine weitere Reinigung wurde das Präzipitat bzw. der Trockenrückstand 2 mal in Diethylether aufgenommen und umkristallisiert und getrocknet. Das Produkt kann ohne andere Verfahrensschritte auch direkt durch Extraktion mit Diethylether aus der wäßrigen Phase gewonnen und entsprechend aufgearbeitet werden.
In den so gewonnenen Präparationen konnte (R)-2,4-DP mittels HPLC nicht mehr nachgewiesen werden (Detektionsgrenze < 0,5 *µ*M). Der Nachweis erfolgte an einer Shimadzu-HPLC Anlage unter isokratischen Bedingungen bei einer Flußrate der mobilen Phase (40% Acetonitril in 60 % Phosphatlösung 130 mM, pH 2,8) von 1 ml/min und einer Nucleosil 5RP18 Säule (250/4) (Knauer, Berlin) bzw. unter Nutzung einer enantioselektiven Säule (Nucleodex-α-PM 200/4; Macherey-Nagel, Düren) mit 70% Methanol in 30% NaH2PO4 (50 mM, pH 3,0) als mobiler Phase.

### Beispiel 3

### Produktion von S-2,4-DP

*D. acidovorans* MC1-S DSM 15377 wurde unter diskontinuierlichen bzw. kontinuierlichen Bedingungen auf (RS)-2,4-DP als alleiniger C/E-Quelle angezogen. Für die weiteren Verfahrensschritte wurde die Biomasse entweder ohne weitere Behandlung eingesetzt bzw. die Biomasse abgetrennt und in einer Konzentration von 0,1 bis 0,5 g/l in ein Medium (mineralisch wie Beispiel 1) mit pH-Werten von 9-9,5 gegeben, welches 5 mM (Raumtemperatur) bis 10 mM (30°C) (RS)-2,4-DP enthielt. Die Suspension wurde unter aeroben Bedingungen entsprechend bei Raumtemperatur bzw. bei 30°C inkubiert. Die Konzentration an (R)-2,4-DP war in einem Zeitraum von 6 bis 24 h in Abhängigkeit von der Biomassekonzentration auf einen über HPLC nicht mehr nachweisbaren Wert abgesunken. Die Suspension wurde nach den in Beispiel 2 aufgezeigten Verfahren aufgearbeitet.

### Beispiel 4

### Produktion von S-2,4-DP

*D. acidovorans* MC1-S DSM 15377 wurde nach einem Batch-Verfahren auf 2 bis 5 g/l Na-Pyruvat angezogen. Als Inokulum dienten Kolonien des auf (RS)-2,4-DP-Agarplatten selektiv gehaltenen Stammes. Nach Erreichen der stationären Phase wurde diese Biomasse durch Zugabe von 0,5 mM (RS)-2,4-DP induziert und dann in ein Medium gegeben, welches die in Beispiel 3 angegebenen Konzentrationen an Biomasse und (RS)-2,4-DP enthielt. Nach entsprechenden Zeiten war die Konzentration an (R)-2,4-DP auf nicht mehr nachweisbare Werte abgesunken.

### Beispiel 5

### Produktion von S-MCPP

Der Stamm MC1-S DSM 15377 wurde wie in Beispiel 2 bei einem D=0,075 h⁻¹ kontinuierlich auf 7,5 mM (RS)-2-(4-Chlor-2-Methylphenoxy)propionat [(RS)-MCPP] kultiviert, der Fermenterablauf unter aseptischen Bedingungen bzw. unter ungeschützten Bedingungen in einer Stickstoffatmosphäre in Portionen steril gesammelt und für 0,25 Stunden aerob bei Raumtemperatur inkubiert oder auf (RS)-MCPP kultivierte Biomasse wurde wie in Beispiel 3 angegeben zur Darstellung von (S)-MCPP in einem diskontinuierlichen Verfahren eingesetzt. Die Kultivierung kann auch auf Pyruvat und anschließender Induktion durch (RS)-MCPP erfolgen wie prinzipiell in Beispiel 4 gezeigt. Entsprechend der Biomassekonzentration wurden in allen Fällen (S)-MCPP Produkte mit über HPLC nicht mehr nachweisbaren Konzentrationen an (R)-MCPP erhalten (Det-ektionsgrenze < 0,5 µM). Die weitere Aufarbeitung des Produktes erfolgte wie in Beispiel 1 für (S)-2,4-DP gezeigt.

### Beispiel 6

### Produktion von (S)-2-(m-Chlorphenoxy)propionat

Die Biomasse wurde wie in Beispiel 2 oder 3 gezeigt angezogen und vor dem weiteren Einsatz mit sterilem Medium gewaschen bzw. wie in Beispiel 4 gezeigt kultiviert und durch Zugabe von (RS)-2-(m-Chlorphenoxypropionat induziert und in beiden Fällen dann in ein Medium mit 5 mM (RS)-2-(m-Chlorphenoxy)propionat gegeben. In Abhängigkeit von der Biomassekonzentration, zugegeben in einem Bereich von 0,1 bis 0,5 g/l erfolgt der komplette Abbau von (R)-2-(m-Chlorphenoxy)propionat bis auf über HPLC nicht mehr nachweisbare Grenzen innerhalb von 6 bis 24 h. In allen Fällen wurden reine S-enantiomere Produkte erhalten.

### Beispiel 7

### Produktion von (S)-2-(4-Chlorphenoxy)propionat

Die Biomasse wurde wie in Beispiel 2 oder 3 gezeigt angezogen und vor dem weiteren Einsatz mit sterilem Medium gewaschen bzw. wie in Beispiel 4 gezeigt kultiviert und durch Zugabe von (RS)-2-(4-Chlorphenoxy)propionat induziert und in beiden Fällen dann in ein Medium mit 5 mM (RS)-2-(4-Chlorphenoxy)propionat gegeben. In Abhängigkeit von der Biomassekonzentration, zugegeben in einem Bereich von 0,1 bis 0,5 g/l erfolgt der komplette Abbau von (R)-2-(4-Chlorphenoxy)propionat bis auf über HPLC nicht mehr nachweisbare Grenzen innerhalb von 6 bis 24 h. In allen Fällen wurden reine S-enantiomere Produkte erhalten.

## Patentansprüche

1. Verfahren zur Gewinnung von Bakterienstämmen *Delftia acidovorans* MC1-S, die zur Produktion hochreiner S-Enantiomeren von (RS)-2-Phenoxypropionsäure-Derivaten geeignet sind,
**dadurch gekennzeichnet, dass**
der Wildtypstamm *Delftia acidovorans* MC1 auf 2,4-D ((2,4-Dichlorphenoxy)essigsäure) als einziger Kohlenstoff- und Energiequelle kultiviert wird und Klone mit ausschließlicher Aktivität für R-Enantiomere von (RS)-2-Phenoxypropionsäure-Derivaten selektiert werden.

2. Verfahren nach Anspruch 1,
**dadurch gekennzeichnet, dass**
die Kultivierung bei Durchflussraten im Bereich von größer Null bis 0,3 h⁻¹ erfolgt.

3. Verfahren nach Anspruch 1 oder 2,
**dadurch gekennzeichnet,**
**dass** zur Kultivierung mindestens eine weitere Kohlenstoff- und Energiequelle vorhanden ist, vorzugsweise Succinat oder Pyruvat.

4. Verfahren nach einem der Ansprüche 1 bis 3,
**dadurch gekennzeichnet,**
**dass** die Kultivierung in einem Nährmedium erfolgt.

5. Verfahren nach einem der Ansprüche 1 bis 4,
**dadurch gekennzeichnet,**
**dass** in definierten zeitlichen Abständen die Durchflussrate von 0,05 h⁻¹ auf 0,3 h⁻¹ gesteigert, anschließend über einen längeren Zeitraum auf dem höchsten Wert konstant gehalten wird, stark wachsende Klone selektiert und dieser Vorgang mehrmals wiederholt wird.

6. Verfahren nach einem der Ansprüche 1 bis 5,
**dadurch gekennzeichnet,**
**dass** eine maximale Wachstumsrate bei einer Durchflussrate von 0,2 bis 0,3 h⁻¹, insbesondere bei 0,24 h⁻¹ erreicht wird.

7. Verfahren nach einem der Ansprüche 1 bis 6,
**dadurch gekennzeichnet,**
**dass** die Kultivierung kontinuierlich über einen Zeitraum von 2 bis 8 Wochen bei einem pH von 8 bis 9, vorzugsweise 8,5, und einer Temperatur von 20 bis 35°C, vorzugsweise 30°C erfolgt.

8. Verfahren nach Anspruch 7,
**dadurch gekennzeichnet,**
**dass** die Kultivierung mit 2,4-D und Succinat über einen Zeitraum von 10 Tagen bei einer Durchflussrate (D) von 0,1 h⁻¹ erfolgt, anschließend mit einem ΔD von 0,06 h⁻¹ und einem Zeitintervall Δt von 1,5 h die Durchflussrate auf 0,24 h⁻¹ gesteigert wird, die Kultur danach 3 Tage konstant gehalten wird, stark wachsende Kolonien selektiert, in Komplexmedium weiter vermehrt und erneut selektiv mit 2,4-D und Succinat kultiviert werden, wobei das Verfahren bis zu dreimal wiederholt wird.

9. Bakterienstämme *Delftia acidovorans* MC1-S, die ausschließlich das S-Enantiomere von (RS)-2-Phenoxypropionsäure-Derivaten produzieren, hergestellt nach einem Verfahren gemäß einem der Ansprüche 1 bis 8.

10. Modifizierte Bakterienstämme *Delftia acidovorans* MC1-S, in denen das Gen für das Enzym (S)-2,4-DP/ α-Ketoglutarat-Dioxygenase (SdpA) abgeschaltet bzw. eliminiert ist.

11. Bakterienstamm *Delftia acidovorans* MC1-S DSM 15377.

12. Verwendung von einem Bakterienstamm *Delftia acidovorans* MC1-S gemäß einem der Ansprüche 9 bis 11 zur Herstellung von hochreinen S-Enantiomeren aus (RS)-2-Phenoxypropionsäure-Derivaten, wobei eine Kultivierung des jeweiligen Stammes kontinuierlich oder diskontinuierlich bei pH-Werten zwischen 6,5 bis 10 und Temperaturen zwischen 15 und 37 °C, erfolgt, die Biomasse abgetrennt und das reine S- Enantiomere aus dem Überstand ausgefällt oder durch Extraktion gewonnen wird.

13. Verwendung nach Anspruch 12,
**dadurch gekennzeichnet, dass**
man den Bakterienstamm *Delftia acidovarans* MC1-S kontinuierlich bei Durchflußraten D von 0,05 h⁻¹ bis 0,20 h⁻¹, pH-Werten zwischen 8 bis 9,5 und Temperaturen zwischen 20 und 30°C, auf einem (RS)-2-Phenoxypropionsäure-Derivat als alleiniger Kohlenstoff- und Energiequelle kultiviert, nach einer Inkubationszeit die Biomasse abtrennt und das verbleibende und nicht metabolisierte (S)-2-Phenoxypropionsäure-Derivat aus dem wässrigen Überstand durch Säurefällung gewinnt.

14. Verwendung nach Anspruch 12,
**dadurch gekennzeichnet, dass**
man den Bakterienstamm *Delftia acidovorans* MC1-S diskontinuierlich auf einem an sich bekannten Nährmedium kultiviert, mit einem betreffenden (RS)-2-Phenoxypropionsäure-Derivat, als Inokulum behandelt, und anschließend die so erzeugte Suspension ohne weitere Aufbereitung oder die Biomasse nach Separation in einer Konzentration von 0,1 bis 0,5 g/l bei einem pH-Wert von 8 - 9,5 in ein Medium überführt, das das (RS)-2-Phenoxypropionsäure-Derivat in einer Konzentration von 5 mM bis 10 mM enthält und kultiviert bis das R-Enantiomere vollständig abgereichert ist.

15. Verwendung nach Anspruch 14,
**dadurch gekennzeichnet, dass**
man das an sich bekannte mineralische Nährmedium und vorzugsweise Pyruvat als Kohlenstoff- und Energiequelle in einer Menge von 2 bis 5 g/l verwendet.

16. Verwendung nach Anspruch 12,
**dadurch gekennzeichnet, dass**
man den Bakterienstamm *Delftia acidovorans* MC1-S kontinuierlich oder diskontinuierlich auf einem (RS)-2-Phenoxypropionsäure-Derivat anzieht, die so gewonnene Biomasse in einer Konzentration von 0,1 bis 0,5 g/l bei einem pH-Wert von 8 - 9,5 in ein Medium überführt, das das (RS)-2-Phenoxypropionsäure-Derivat in einer Konzentration von 5 mM bis 10 mM enthält und kultiviert bis das R-Enantiomere vollständig abgereichert ist.

17. Verwendung nach einem der Ansprüche 12 bis 16,
**dadurch gekennzeichnet, dass**
als (RS)-2-Phenoxypropionsäure-Derivate
(RS)-2-(2,4-Dichlorphenoxy)propionsäure [(RS)-2,4-DP],
(RS)-2-(4-Chlor-2-methylphenoxy)propionsäure [(RS)-MCPP],
(RS)-2-(m-Chlorphenoxy)propionsäure und
(RS)-2-(4-Chlorphenoxy)propionsäure verwendet werden.

18. Verwendung von einem Bakterienstamm *Delftia acidovorans* MC1-S gemäß einem der Ansprüche 9 bis 11 zur Verbesserung und Reinigung kommerzieller S- Enantiomere-Produkte.

19. Verwendung von einem Bakterienstamm *Delftia acidovorans* MC1-S gemäß einem der Ansprüche 9 bis 11 zur Eliminierung bzw. Abreicherung des R-Enantiomers aus racemischen Gemischen durch produktiven Abbau im *bulk production* Maßstab.

## Claims

1. A method of obtaining bacterial strains *Delftia acidovorans* MC1-S suitable for the production of high-purity S-enantiomers of (RS)-2-phenoxypropionic acid derivatives,
**characterized in that**
the wild type strain *Delftia acidovorans* MC1 is cultured on 2,4-D ((2,4-dichlorophenoxy)acetic acid) as the only source of carbon and energy, and clones solely active on R-enantiomers of (RS)-2-phenoxypropionic acid derivatives are selected.

2. The method according to claim 1,
**characterized in that**
culturing is effected at flow rates in a range of from greater than zero to 0.3 h⁻¹.

3. The method according to claim 1 or 2,
**characterized in that**
at least one additional source of carbon and energy is present for culturing, preferably succinate or pyruvate.

4. The method according to any of claims 1 to 3,
**characterized in that**
culturing is effected in a nutrient medium.

5. The method according to any of claims 1 to 4,
**characterized in that**
the flow rate is increased from 0.05 h⁻¹ to 0.3 h⁻¹ at well-defined time intervals, subsequently kept constant at the maximum value for a prolonged period of time, strongly growing clones are selected, and this operation is repeated several times.

6. The method according to any of claims 1 to 5,
**characterized in that**
maximum growth rate is achieved at a flow rate of from 0.2 to 0.3 h⁻¹, especially at 0.24 h⁻¹.

7. The method according to any of claims 1 to 6,
**characterized in that**
culturing is effected continuously for a period of from 2 to 8 weeks at a pH of from 8 to 9, preferably 8.5, and at a temperature of from 20 to 35°C, preferably 30°C.

8. The method according to claim 7,
**characterized in that**
culturing with 2,4-D and succinate is effected for a period of 10 days at a flow rate (D) of 0.1 h⁻¹, the flow rate is subsequently increased to 0.24 h⁻¹ at a ΔD of 0.06 h⁻¹ and a time interval Δt of 1.5 h, the culture then is kept constant for 3 days, strongly growing colonies are selected, further grown in complex medium and selectively re-cultured with 2,4-D and succinate, and this operation is repeated up to three times.

9. Bacterial strains *Delftia acidovorans* MC1-S, solely producing the S-enantiomers of (RS)-2-phenoxypropionic acid derivatives, said strains being produced using a method according to any of claims 1 to 8.

10. Modified bacterial strains *Delftia acidovorans* MC1-S, wherein the gene for the enzyme (S)-2,4-DP/α-ketoglutarate dioxygenase (SdpA) is switched off or eliminated.

11. The bacterial strain *Delftia acidovorans* MC1-S DSM 15377.

12. Use of a bacterial strain *Delftia acidovorans* MC1-S according to any of claims 9 to 11 in the production of high-purity S-enantiomers of (RS)-2-phenoxypropionic acid derivatives, wherein culturing of the respective strain is effected continuously or discontinuously at pH values between 6.5 and 10 and temperatures between 15 and 37°C, the biomass is removed, and the pure S-enantiomer is precipitated from the supernatant or obtained by extraction.

13. The use according to claim 12,
**characterized in that**
the bacterial strain *Delftia acidovorans* MC1-S is cultured continuously at flow rates D of from 0.05 h⁻¹ to 0.20 h⁻¹, pH values between 8 and 9.5 and temperatures between 20 and 30°C on an (RS)-2-phenoxypropionic acid derivative as the only source of carbon and energy, the biomass is removed subsequent to an incubation time, and the remaining non-metabolized (S)-2-phenoxypropionic acid derivative is obtained from the aqueous supernatant by acid precipitation.

14. The use according to claim 12,
**characterized in that**
the bacterial strain *Delftia acidovorans* MC1-S is cultured discontinuously on a per se known nutrient medium, treated with a relevant (RS)-2-phenoxypropionic acid derivative as inoculum, and thereafter, the suspension thus generated, without further treatment, or the biomass, after separation, is transferred at a concentration of from 0.1 to 0.5 g/l at a pH value of 8 to 9.5 into a medium containing said (RS)-2-phenoxypropionic acid derivative at a concentration of from 5 mM to 10 mM, and culturing is effected until the R-enantiomer is completely depleted.

15. The use according to claim 14,
**characterized in that**
the per se known mineral nutrient medium and preferably pyruvate as source of carbon and energy in an amount of from 2 to 5 g/l are used.

16. The use according to claim 12,
**characterized in that**
the bacterial strain *Delftia acidovorans* MC1-S is pre-cultured continuously or discontinuously on an (RS)-2-phenoxypropionic acid derivative, the biomass thus obtained is transferred at a concentration of from 0.1 to 0.5 g/l at a pH value of 8 to 9.5 into a medium containing said (RS)-2-phenoxypropionic acid derivative at a concentration of from 5 mM to 10 mM, and culturing is effected until the R-enantiomer is completely depleted.

17. The use according to any of claims 12 to 16,
**characterized in that**
(RS)-2-(2,4-dichlorophenoxy)propionic acid [(RS)-2,4-DP], (RS)-2-(4-chloro-2-methylphenoxy)propionic acid [(RS)-MCPP], (RS)-2-(m-chlorophenoxy)propionic acid and (RS)-2-(4-chlorophenoxy)propionic acid are used as (RS)-2-phenoxypropionic acid derivatives.

18. Use of a bacterial strain *Delftia acidovorans* MC1-S according to any of claims 9 to 11 for improving and purifying commercial S-enantiomer products.

19. Use of a bacterial strain *Delftia acidovorans* MC1-S according to any of claims 9 to 11 for eliminating or depleting an R-enantiomer from racemic mixtures by productive degradation on a bulk production scale.

## Revendications

1. Procédé d'obtention de souches bactériennes *Delftia acidovorans* MC1-S, qui conviennent à la production d'énantiomères S ultrapurs de dérivés d'acide (RS)-2-phénoxypropionique,
**caractérisé en ce qu'**on
cultive la souche de type sauvage *Delftia acidovorans* MC1 sur de l'acide 2,4-D (acide (2,4-dichlorophénoxy) acétique) à titre de source unique de carbone et d'énergie et qu'on sélectionne des clones exclusivement actifs vis à vis des énantiomères R des dérivés d'acide (RS)-2-phénoxypropionique.

2. Procédé selon la revendication 1,
**caractérisé en ce**
**qu'**on effectue la culture à des débits compris dans une plage allant d'un débit supérieur à zéro à 0,3 h⁻¹.

3. Procédé selon la revendication 1 ou 2,
**caractérisé en ce**
**qu'**il y au moins pour la culture une autre source de carbone et d'énergie, de préférence du succinate ou du pyruvate.

4. Procédé selon l'une des revendications 1 à 3,
**caractérisé en ce**
**qu'**on effectue la culture dans un milieu nutritif.

5. Procédé selon une des revendications 1 à 4,
**caractérisé en ce**
**qu'**on augmente dans des espaces de temps définis le débit de 0,05 h⁻¹ à 0,3 h⁻¹, puis qu'on le maintient constant pendant une période prolongée à la valeur maximale, qu'on sélectionne les clones qui ont une croissance forte et qu'on répète ce processus plusieurs fois.

6. Procédé selon une des revendications 1 à 5,
**caractérisé en ce**
**qu'**on obtient un taux de croissance maximal à un débit de 0,2 à 0,3 h⁻¹, notamment à 0,24 h⁻¹.

7. Procédé selon une des revendications 1 à 6,
**caractérisé en ce**
**qu'**on effectue la culture en continu pendant une période de 2 à 8 semaines, à un pH de 8 à 9, de préférence à 8,5, à une température de 20 à 35°C, de préférence à 30°C.

8. Procédé selon la revendication 7,
**caractérisé en ce**
**qu'**on effectue la culture avec de l'acide 2,4-D et du succinate pendant une période de 10 jours à un débit (D) de 0,1 h⁻¹, puis on augmente par pas de ΔD = 0,06 h⁻¹ le débit pour atteindre en Δt = 1,5 h un débit de 0,24 h-¹, on maintient ensuite la culture à ce débit constant pendant 3 jours, on sélectionne les colonies à forte croissance, on les fait proliférer encore dans un milieu complexe, et on effectue la culture à nouveau sélectivement avec de l'acide 2,4-D et du succinate, en répétant le procédé jusqu'à trois fois.

9. Souches bactériennes *Delftia acidovorans* MC1-S, qui produisent exclusivement l'énantiomère S des dérivés de l'acide (RS)-2-phénoxypropionique, préparées selon un procédé d'après une des revendications 1 à 8.

10. Souches bactériennes modifiées *Delftia acidovorans* MC1-S, dans lesquelles le gène pour l'enzyme (S)-2,4-DP/α-cétoglutarate-dioxygénase (SdpA) est scindé ou éliminé.

11. Souche bactérienne *Delftia acidovorans* MC1-S DSM 15377.

12. Utilisation d'une souche bactérienne *Delftia acidovorans* MC1-S selon une des revendications 9 à 11 pour préparer des énantiomères S ultrapurs de dérivés d'acide (RS)-2-phénoxypropionique, moyennant quoi on effectue une culture de la souche respective en continu ou de manière discontinue à un pH compris entre 6,5 et 10 et à des températures comprises entre 15 et 37°C, on sépare la biomasse et on précipite l'énantiomère S pur dans le surnageant ou on l'obtient par extraction.

13. Utilisation selon la revendication 12,
**caractérisé en ce**
**qu'**on cultive la souche bactérienne *Delftia acidovorans* MC1-S en continu à des débits D de 0,05 h⁻¹ à 0,20 h⁻¹, à des pH de 8 à 9,5 et à des températures de 20 à 30°C, sur un dérivé d'acide (RS)-2-phénoxypropionique à titre d'unique source de carbone et d'énergie, on sépare la biomasse après une durée d'incubation et on obtient le dérivé d'acide (S)-2-phénoxypropionique restant et non métabolisé à partir du surnageant aqueux par précipitation acide.

14. Utilisation selon la revendication 12,
**caractérisée en ce**
**qu'**on cultive la souche bactérienne *Delftia acidovorans* MC1-S de manière discontinue dans un milieu nutritif connu en soi, qu'on traite avec un dérivé d'acide (RS)-2-phénoxypropionique concerné, à titre d'inoculum, et qu'on transfère ensuite la suspension ainsi préparée sans autre traitement ou la biomasse après séparation à une concentration de 0,1 à 0,5 g/l à un pH de 8 - 9,5 dans un milieu, qui contient le dérivé d'acide (RS)-2-phénoxypropionique à une concentration de 5 mM à 10 mM et on cultive jusqu'à ce que l'énantiomère R soit complètement appauvri.

15. Utilisation selon la revendication 14,
**caractérisée en ce**
**qu'**on utilise le milieu nutritif minéralisé connu en soi et de préférence du pyruvate à titre de source de carbone et d'énergie en une quantité de 2 à 5 g/l.

16. Utilisation selon la revendication 12,
**caractérisée en ce**
**qu'**on précultive la souche bactérienne *Delftia acidovorans* MC1-S sur un dérivé d'acide (RS)-2-phénoxypropionique, on transfère la biomasse ainsi obtenue à une concentration de 0,1 à 0,5 g/l, à un pH de 8 - 9,5 dans un milieu, qui contient le dérivé d'acide (RS)-2-phénoxypropionique à une concentration de 5 mM à 10 mM et on réalise la culture jusqu'à ce que l'énantiomère R soit complètement appauvri.

17. Utilisation selon une des revendications 12 à 16,
**caractérisée en ce**
**qu'**on utilise, en tant que dérivés d'acide (RS)-2-phénoxypropionique, de l'acide (RS)-2-(2,4-dichlorophénoxy)-propionique [(RS)-2,4-DP], de l'acide (RS)-2-(4-chloro-2-méthylphénoxy)propionique [(RS)-MCPP], de l'acide (RS)-2-(m-chlorophénoxy)propionique et de l'acide (RS)-2-(4-chlorophénoxy)propionique.

18. Utilisation d'une souche bactérienne *Delftia acidovorans* MC1-S selon une des revendications 9 à 11 pour améliorer et purifier les produits énantiomères S à des fins commerciales.

19. Utilisation d'une souche bactérienne *Delftia acidovorans* MC1-S selon une des revendications 9 à 11 pour éliminer ou appauvrir l'énantiomère R dans des mélanges racémiques par décomposition productive à une échelle de production en grande quantité.
